# EUROPEAN PATENT SPECIFICATION

(11) **EP 2 057 992 B1**
(45) Date of publication and mention of the grant of the patent: **27.02.2013**
(21) Application number: 07792490.0
(22) Date of filing: 14.08.2007
(51) Int. Cl.: A61K 35/74, A61P 13/12

(54) **RENAL-FUNCTION-AMELIORATING AGENT**
MITTEL ZUR VERBESSERUNG DER NIERENFUNKTION
AGENT D'AMÉLIORATION DE LA FONCTION RÉNALE

(30) Priority: 21.08.2006 JP 2006224670
(43) Date of publication of application: 13.05.2009
(73) Proprietor: Calpis Co., Ltd., Shibuya-ku, Tokyo 150-0022 (JP)
(72) Inventor: SUZUKI, Hiromi, Sagamihara-shi Kanagawa 229-0006 (JP); FUJIWARA, Shigeru, Sagamihara-shi Kanagawa 229-0006 (JP)
(74) Representative: Nemec, Harald
(86) International application number: PCT/JP2007/065847
(87) International publication number: WO 2008/023607

(56) References cited:
- WO-A1-2005/032591
- WO-A2-2005/019417
- JP-A- 54 011 233
- JP-A- 2005 532 294
- SAVICA V ET AL: "Nutritional therapy in chronic kidney disease", NUTRITION IN CLINICAL CARE, BLACKWELL PUBLISHING, INC., MALDEN, MA, US, vol. 8, no. 2, 1 April 2005 (2005-04-01), pages 70-76, XP009086900, ISSN: 1096-6781
- SUZUKI H. ET AL.: 'Effect of Bacillus subtilis C-3102 Intakes on the Composition and Metabolic Activity of Fecal Microflora of Humans' JOURNAL OF INTESTINAL MICROBIOLOGY vol. 18, 2004, pages 93 - 99, XP003021302

### SEQUENCE LISTING

<110> Calpis Co. Ltd.
<120> RENAL-FUNCTION-AMELIORATING AGENT
<130> PCP-9005W0
<150> JP 2006-224670
   <151> 2006-08-21
<160> 2
<170> PatentIn version 3.1
<210> 1
   <211> 30
   <212> DNA
   <213> Bacillus subtilis
<400> 1
   gccccgcaca tacgaaaaga ctggctgaaa 30
<210> 2
   <211> 30
   <212> DNA
   <213> Bacillus subtilis
<400> 2
   ggatcccacg ttgtgattaa aagcagcgat 30

## Description

### TECHNICAL FIELD

The present invention relates to a renal function-ameliorating agent which comprises as an active ingredient cells or a culture of a bacterium of the genus *Bacillus,* particularly *Bacillus subtilis* C-3102, and which has a serum creatinine-lowering action.

### BACKGROUND

Kidney diseases (excluding, for example, acute nephritis) are diseases which continue throughout the patient's life. Many such diseases progress at a certain frequency, sometimes leading ultimately to terminal renal failure (a state that necessitates dialysis treatment). Because the kidneys have a substantial reserve capacity, chronic renal disorders are substantially asymptotic until renal function falls to 20% or below. It is often the case that when symptoms of some sort have appeared, the kidney disease has already advanced to a stage where terminal renal failure arises and dialysis is required.

Creatinine is a non-proteinous nitrogen compound which is produced within the muscles from creatinine and is as an excellent indicator of renal function (glomerular filtration rate) that is not influenced by extrinsic factors such as the diet. As the kidney disease progresses and renal function falls below 50% of the normal level, the serum creatinine begins to rise. At this stage, the primary approach is dietary therapy where protein and salt intake is restricted, with adjuvant pharmacotherapy. However, when renal function drops to a level of 20 to 30% or less, renal failure arises, at which point serum creatinine levels will not normalize with a dietary therapy. At a renal function of 5 to 10% or below, renal dialysis is necessary.

Currently the number of chronic dialysis patients is reported to be about 200,000, and more than 30,000 new patients start dialysis every year. In the case of chronic nephropathy, the goal is to delay the progress of the disease and enable renal function to be maintained as long as possible, but there is no mode of treatment that cures the disease as such. The current therapy is to slow the progression of disease through dietary therapy and pharmacotherapy for renal failure, and thereby maintain the residual renal function as long as possible.

The glomerular filtration rate and renal blood flow rate which serve as indicators of renal function are known to decrease linearly with age. The decline in renal function is believed to be one of the major factors in human aging.

Accordingly, there exists a desire for drugs and food products with a creatinine-lowering activity which have no side effects as in pharmacotherapy and is easy to ingest for a long time.

### DISCLOSURE OF THE INVENTION

The object of the invention is to provide an agent useful for treating and preventing renal diseases.

The present invention provides a renal function-ameliorating agent comprising as an active ingredient cells or a culture of a bacterium belonging to the genus *Bacillus.* The invention also provides a serum creatinine-lowering agent comprising as an active ingredient cells or a culture of a bacterium belonging to the genus *Bacillus.* The bacterium belonging to the genus *Bacillus* is preferably *Bacillus subtills*, and more preferably *Bacillus subtilis* C-3102 (FERM BP-1096).

### BRIEF DESCRIPTION OF THE DRAWINGS

FIG. 1 shows the restriction enzyme NotI or SfiI digestion patterns of genomic DNA from *Bacillus subtilis* C-3102.
FIG. 2 shows the change over time in serum creatinine concentration in subjects who ingested the renal function-ameliorating agent of the present invention.

### PREFERRED EMBODIMENTS OF THE INTENTION

Bacteria of the genus *Bacillus* (e.g., *Bacillus subtilis*) have long been closely associated with the dietary habits of man. Although ample information exists on the functionality of this organism, it has not previously been reported to have a serum creatinine-lowering action or a renal function-ameliorating effect.

The renal function-ameliorating agent and serum creatinine-lowering agent of the present invention are characterized by comprising as an active ingredient cells or a culture of a bacterium of the genus *Bacillus,* and preferably cells or a culture of *Bacillus subtilis.* The bacteriological characteristics of *Bacillus subtilis* are described in, for example, Bergey's Manual of Bacteriology, Vol. 11 (1986), and include the following.
(1) Gram positive
(2) Forms oval spores
(3) Rod-shaped
(4) Motile
(5) Aerobic
(6) Catalase: positive
(7) Growth at 50°C: +
(8) Growth at pH 5.7: +
(9) Utilization of citrate: +
(10) Acid production from the sugars arabinose, glucose, xylose, mannitol: +
(11) VP reaction: +
(12) Starch hydrolysis: +
(13) Nitrate reduction: +
(14) Indole production: -
(15) Gelatin hydrolysis: +
(16) Casein hydrolysis: +
(17) Film formation in liquid medium: +
(18) Curdling of milk: -
(19) Peptonization of milk; +

The *Bacillus subtilis* used in the renal function-ameliorating agent and serum creatinine-lowering agent of the invention may include, for example, *Bacillus subtilis* C-3102 (deposited on December 25, 1985 at Japan's National Institute of Bioscience and Human Technology under accession number FERM BP-1096). Soybean cultures of *Bacillus subtilis* C-3102 have a number of desirable effects in livestock, such as improving the intestinal flora, somatic growth, preventing infection, increasing eggshell strength, enhancing meat quality and ameliorating fecal odors, and are used as feed additives (Japanese Patent Publication No. H4-24022). The beneficial effects of this strain on human health include regulating intestinal function and decreasing the products of intestinal putrefaction ( Suzuki H. et al., Journal of Intestinal Microbiology, Vol. 18, No. 2, 93-99 (2004)).

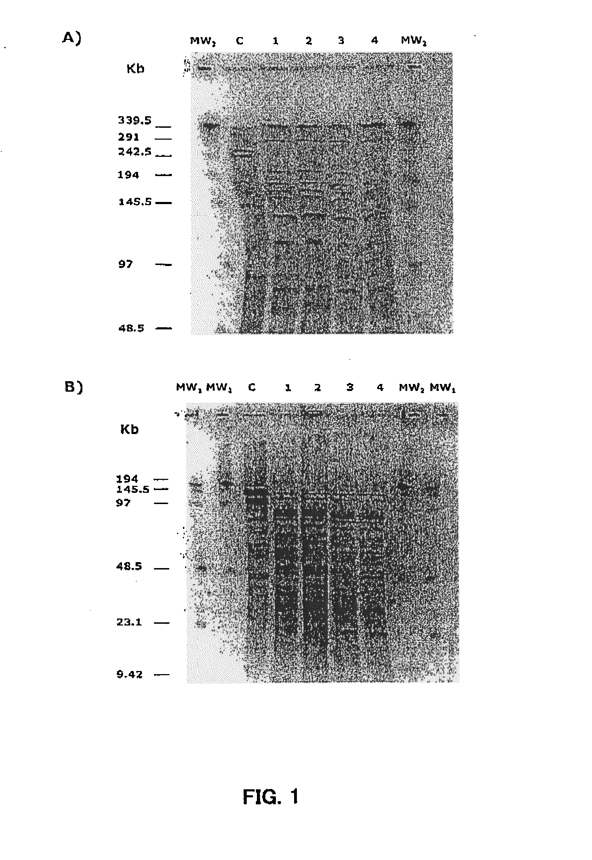

With *Bacillus subtilis* C-3102, an approximately 700 bps fragment is amplified by PCR using the PCR primers of SEQ ID NOs:1 and 2 below. In other *Bacillus subtilis* strains, no amplification is observed with these PCR primers. The approximately 700 bps fragment amplified with the *Bacillus subtilis* C-3102 genome as the template has no homology with the amylase sequence. This clearly distinguishes the C-3102 strain from other *Bacillus subtilis* strains.
Sequence 1: 5'-GCCCCGCACATACGAAAAGACTGGCTGAAA-3' (SEQ ID NO: 1)
Sequence 2: 5'-GGATCCCACGTTGTGATTAAAAGCAGCGAT-3' (SEQ ID NO: 2)
*Bacillus subtilis* C-3102 also has the following characteristics.
(1) Lacks plasmid DNA.
(2) The digestion pattern obtained from digestion of the genomic DNA with the restriction enzyme NotI or SfiI. and separation by agarose electrophoresis is as shown in FIG. 1.
(3) Produces antibacterial substances to *B. cerous*.
(4) Lacks resistance to ampicillin, chloramphenicol, ciprofloxacin, erythromycin, gentamicin, kanamycin, linezolid, quinupristin/dalfopristin, rifampin, streptomycin, tetracycline, trimethoprim and vancomycin (in each case, the minimum inhibitory concentration is from 0.03 to 4 µg/mL).

*Bacillus subtilis* may be cultured using a liquid or solid culture medium commonly used to grow microorganisms, which contains, for example, a carbon source, a nitrogen source and inorganic substances. The carbon source may be any of those can be utilized by *Bacillus subtilis,* such as glucose, fructose, sucrose, starch or molasses. Examples of nitrogen sources that may be used include peptones, casein hydrolyzates, meat extracts, and ammonium sulfate. In addition, phosphoric acids and salts thereof, such as potassium, magnesium, calcium, sodium, iron and manganese salts, and also vitamins, amino acids and surfactants may be added as needed. In addition to such synthetic media. *Bacillus subtilis* may also be cultured using substances derived from natural products, such as soybean oil meal. Cultivation is preferably carried out under aerobic conditions. Preferred examples of the culture apparatus include apparatuses for aeration spinner liquid culturing with a jar fermentor, tray-type solid culture apparatuses, and automated *koji*-making culture apparatuses. The culture temperature is preferably from 20 to 50°C, and more preferably from 30 to 45°C; the culture period is from 12 hours to 7 days; and the initial pH is preferably from 5 to 9, and more preferably from 6 to 8.

The culture thus obtained contains *Bacillus subtilis* cells, medium and fermentation products. The culture may be used directly without processing as a renal function-ameliorating agent or a serum creatinine-lowering agent. Alternatively, the culture may be concentrated and used. Excipients and other ingredients may be added to the culture or the concentrated culture for use as a preparation in the form of dry powder, granules or tablets. Cells isolated from the culture, the culture free from the cells, or the culture containing the cells may be used in the present invention. In an especially preferred embodiment, *Bacillus subtilis* is cultured using substances of natural origin which are suitable for use as foods, such as soybean oil meal, boiled soybeans, boiled adzuki beans, boiled rice, rice boiled with barley, wheat bran, boiled corn, and other grains, and is directly mixed into a food product without separating the cells from the culture.

The renal function-ameliorating agent and serum creatinine-lowering agent of the invention may be taken in the form of, for example, a liquid, powder, granules or tablets, or may be mixed as a food additive into beverage and food products and ingested. Illustrative examples of beverage and food products include drinks, candies and sweets, pastes, bread, processed fish and meat products, and dairy products. The renal function-ameliorating agent and serum creatinine-lowering agent of the invention may be added to these various food materials and furnished as health drinks, health foods, or nutraceuticals having health-promoting benefits.

As described above, the present invention provides a renal function-ameliorating agent with a serum creatinine-lowering activity, which comprises as an active ingredient *Bacillus subtilis* cells or a culture thereof.
The *Bacillus subtilis* serving as the active ingredient in the present invention is effective in very small amounts and in a short period of time. It has an excellent preservability and acid resistance, easily reaches to and grows within the large intestine, and can be expected to have a sustained serum creatinine-lowering effect.

The entire contents of all patents and reference documents cited in this specification are incorporated herein by reference. Also, the entire contents of the specification and drawings of Japanese Patent Application No. 2006-224670, which serves as the basis for the priority claim of the present application, are incorporated herein by reference.

The present invention is described in more detail below by way of a working example, although the scope of the invention is not limited by the examples.

### Example 1

In the following working example, *Bacillus subtilis* C-3102 (deposited on December 25, 1985 at Japan's National Institute of Bioscience and Human Technology under accession number FERM BP-1096) was used as an example of a bacterium belonging to the genus *Bacillus*.

Five kilograms of tap water was added to 5 kg of granulated commercial soybean oil meal, and the mixture was sterilized at 121°C for 120 minutes, then inoculated with a culture broth of *Bacillus subtilis* C-3102 (FERM BP-1096) that had been pre-cultured. The resulting mixture was cultured at 37°C for 40 hours to produce a soybean culture of *Bacillus subtilis* C-3102. The resulting culture was dry ground, blended with other ingredients shown in the table below, and 500 mg tablets (each containing 3×10⁹ *Bacillus subtilis* spores) were prepared. Table 1-1 shows the nutrients contained within the tablets, and Table 1-2 shows the composition of the tablets.

**Table 1**

| Nutrient analysis of tablets (per 100 g) | | |
|---|---|---|
| Nutrients | | Values |
| Protein | g/100 g | 4.1 |
| Lipids | g/100 g | 1.4 |
| Ash | g/100 g | 18 |
| Carbohydrates | g/100 g | 74.6 |
| Energy | Kcal/100 g | 327 |
| Sodium | Mg/100 g | 20.2 |

**Tablet composition**

| Ingredient | Content (%) |
|---|---|
| Powdered sugar | 64.10% |
| Eggshell calcium | 22.00% |
| Glucose | 6.00% |
| *Bacillus subtilis* C-3102 soybean culture | 5.60% |
| Sucrose ester | 1.00% |
| Shellac | 0.60% |
| Gum arabic powder | 0.35% |
| Carnauba wax | 0.35% |

### Example 2

### Serum Creatinine Suppression Test (Human Ingestion Test)

### Procedure:

Ten healthy males and females (5 women and 5 men) from 25 to 40 years of age were selected as the subjects. The selection criteria are shown in Table 2. Individuals taking or ingesting specific drugs or foods for specified health use were excluded.

**Table 2**

| | |
|---|---|
| Ten individuals (5 men and 5 women) who satisfied the following criteria and did not meet the exclusion criteria were chosen as subjects. | |

| Selection criteria (criteria for selecting the subjects) | |
|---|---|
| (1) | Individuals from 25 to 40 years of age. |
| (2) | Individuals not on any medication and free of apparent illness (TG, T-cho, BS, blood pressure, constipation, etc.). |
| a) | Neutral fat < 250 mg/dL. |
| b) | Total cholesterol < 240 mg/dL. |
| c) | Fasting blood sugar < 126 mg/dL. |
| d) | GOT < 50 IU/L; GPT < 50 IU/L; γ-GTP < 100 IU/L. |
| e) | Systolic blood pressure < 140 mmHg; Diastolic blood pressure < 90 mmHg. |
| (3) | Individuals with a relatively constant diet and level of exercise. |
| (4) | Individuals receiving regular examinations at a medical facility. |
| (5) | Individuals who do not manifest an anaphylactic reaction to ingestion of the food under study (fermented soybean culture). |
| (6) | Individuals capable of limiting their intake of alcoholic beverages (to not more than the equivalent of 500 mL of beer per day). |
| (7) | Individuals able to maintain a constant daily lifestyle during the course of the test. |
| (8) | Individuals able to keep a diary (to record ingestion of the agent under study, exercise, diet and body weight) throughout the period of the test. |
| (9) | Individuals who, prior to the start of the test, have given their consent to participate in the test and have impressed their personal seal to or signed the consent form and entered the date. |

The subjects orally ingested one 500 mg tablet (a *Bacillus subtilis* C-3102 soybean culture tablet containing 3×10⁹ spores) daily, whenever possible after breakfast, for a period of four weeks. Fasting blood samples (no eating or drinking after 9 PM the night before; blood drawn at a fixed time in the morning) were collected before the start of ingestion and after one week, two weeks, three weeks and four weeks from the start of ingestion, and the serum creatinine level in each sample was measured. The test schedule is shown in Table 3.

**Table 3 Test schedule (test items/period)**

| | Period of ingestion (4 weeks) | | | | |
|---|---|---|---|---|---|
| Test Items/Period (week) | Start | 1 week | 2 weeks | 3 weeks | 4 weeks |
| | around Sep 22 | around Sep 29 | around Oct 6 | around Oct 13 | around Oct 20 |
| Body Mass Index + Cardiovascular Exam Height (initial exam}, weight, body fat percentage (BI method), blood pressure, pulse, temperature | ○ | ○ | ○ | ○ | ○ |
| Blood Biochemistry (1) TG, T-cho, AG ratio, total bilirubin, fasting blood sugar, GOT, GPT, AL-P, γ-GTP, amylase, LDL-cho, HDL-cho, LDH, total protein, albumin, UA, BUN, creatinine, ZTT, Na, Cl, K, Ca, ionized calcium, P, Mg, Fe | ○ | ○ | ○ | ○ | ○ |
| Blood Biochemistry (2) PT (prothrombin), hepaplastin, HbAlc, TT (Thrombotest), fibrinogen, APTT (thromboplastin time), vitamin K fraction | ○ | ○ | ○ | ○ | ○ |
| General Blood Tests WBC, RBC, Hb, Ht, MCV, MCH, MCHC, platelets | ○ | ○ | ○ | ○ | ○ |
| General Urine Tests Sugars, protein, urobilinogen, sediment (performed when protein-positive) | ○ | ○ | ○ | ○ | ○ |
| Patient Interview by Doctor: Examination Checked for subjective symptoms and adverse events, checked daily journal and provided guidance | ○ | ○ | ○ | ○ | ○ |

| | | | | | |
|---|---|---|---|---|---|
| ○: tested | | | | | |

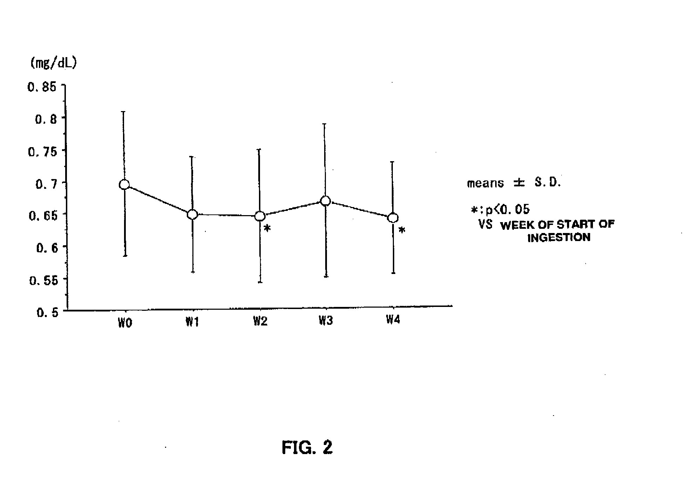

The results of the above test are shown in FIG. 2. The creatinine level over time was 0.696±0.113 mg/dL at the start of ingestion, 0.648±0.0893 mg/dL after one week of ingestion, 0.644±0.1032 mg/dL after two weeks of ingestion. 0.667±0.1179 mg/dL after three weeks of ingestion, and 0.639±0.0862 mg/dL after four weeks of ingestion. A one-way analysis of variance for repeated measurements based on the ingestion period revealed a significant difference (p<0.05). As a result of multiple comparison, significant differences were observed between the start of ingestion and after two weeks of ingestion (p<0.05) and between the start of ingestion and after four weeks of ingestion (p<0.05). From the above results, it was apparent that soybean cultures of *Bacillus subtilis* C-3102 have a serum creatinine-lowering effect.

### INDUSTRIAL APPLICABILITY

The renal function-ameliorating agent of the present invention is able to lower the serum creatinine concentration in humans, and is thus useful as an agent for preventing and ameliorating renal diseases.

## Claims

1. A composition comprising a culture of *Bacillus subtilis* C-3102, deposited under number FERM BP-1096, for use in a method of preventing and ameliorating renal diseases.

2. A composition comprising as an active ingredient spores of *Bacillus subtilis* C-3102, deposited under number FERM BP-1096, for use in a method of preventing and ameliorating renal diseases.

## Patentansprüche

1. Zusammensetzung, umfassend eine Kultur von *Bacillus subtilis* C-3102, hinterlegt unter der Nummer FERM BP-1096, zur Verwendung in einem Verfahren zur Verhütung und Linderung von Nierenerkrankungen.

2. Zusammensetzung, welche als Wirkstoff Sporen von *Bacillus subtilis* C-3102, hinterlegt unter der Nummer FERM BP-1096, umfasst, zur Verwendung in einem Verfahren zur Verhütung und Linderung von Nierenerkrankungen.

## Revendications

1. Composition comprenant une culture de *Bacillus subtilis* C-3102, déposée sous le numéro FERM BP-1096, à utiliser pour prévenir contre les maladies rénales et pour les traiter.

2. Composition comprenant comme ingrédient actif des spores de *Bacillus subtilis* C-3102, déposée sous le numéro FERM BP-1096, à utiliser pour prévenir contre les maladies rénales et pour les traiter.
